# EUROPEAN PATENT APPLICATION

(11) **EP 2 243 494 A1**
(43) Date of publication of application: **27.10.2010**
(21) Application number: 09005672.2
(22) Date of filing: 22.04.2009
(51) Int. Cl.: A61K 45/06

(54) **Pharmaceutical composition, comprising a steroid-dehydrogenase-reductase inhibitor, and a mineralocorticoid receptor antagonist.**

(71) Applicant: OntoChem GmbH, 06120 Halle / Saale (DE)
(72) Inventor: Wilckens, Thomas, 80796 München (DE); Weber, Lutz, 82110 Germering (DE)
(74) Representative: Forstmeyer, Dietmar

(57) **Abstract**

A treatment using a hydroxysteroid dehydrogenase reductase inhibitor combined with a mineralocorticoid receptor antagonist is provided which overcomes the problem that manipulating cortisol levels by a single compound therapy induces other pathologies or side effects, while the original condition that required treatment is ameliorated.

## Description

A treatment using a hydroxysteroid dehydrogenase reductase inhibitor combined with a mineralocorticoid receptor antagonist is provided which overcomes the problem that manipulating cortisol levels by a single compound therapy induces other pathologies or side effects, while the original condition that required treatment is ameliorated.

Endogenous glucocorticoids as well as mineralocorticoids play an essential role in the maintenance of a plethora of physiological functions. Disruption of the mechanisms that control access of these steroid hormones to their connate receptors result in undesired effects, similar to those that are well described for the use of exogenously applied steroid hormone receptor ligands as well a modulators of endogenous steroid availability for ligand binding. In order to minimize undesired effects concepts and relevant tools are needed to ameliorate these undesired effects. The invention provides a novel therapeutic solution by combining specific therapeutic molecules with each other in order to prevent or reverse undesired effects induced by the manipulation of endogenous steroid hormone effects as well effects induced by exogenous steroid hormone mimetics or modulators.

Glucocorticoids were named for their ability to promote the conversion of proteins and lipids into glucose during the stress-induced activation of the hypothalamic-pituitary-adrenal (HPA) axis. In addition to this role in intermediary metabolism, glucocorticoids regulate numerous tissue-specific activities including immune function, the inflammatory response, embryogenesis, behaviour, bone turn-over and cell proliferation and survival. The pleiotropic effects of glucocorticoids provide an abundance of opportunities for pharmacological exploitation. Indeed, synthetic glucocorticoid agonists are one of the most widely prescribed classes of drugs worldwide and are indispensable in the treatment of autoimmune diseases, inflammatory disorders and cancer. However, the physiological and supraphysiological response to glucocorticoids is not uniform, differing not only among individuals but also within tissues of the same individual. The individual- and tissue-specific nature of the glucocorticoid response can be illustrated by examining the effect of glucocorticoids in the treatment of acute lymphoblastic leukemia (ALL). Glucocorticoids are routinely included in chemotherapeutic regimens for ALL because of their ability to induce lymphocyte apoptosis, thereby inhibiting the expansion of cells that have escaped the normal constraints of the cell cycle. Unfortunately, subpopulations of individuals undergoing glucocorticoid based therapy harbour cells that are resistant to glucocorticoid-induced apoptosis and fail to respond to treatment. Thus, glucocorticoids do not offer a therapeutic advantage in all cases of ALL. Although glucocorticoids promote lymphocyte apoptosis, they protect against apoptosis in the endometrium, ovarian follicle, hepatocyte, fibroblast and mammary epithelium. Due to this tissue specificity, glucocorticoids are not useful in treating all malignancies. The therapeutic response to glucocorticoid therapy is further complicated by the fact that individuals undergoing chronic treatment commonly develop dose-limiting side-effects, such as an increased risk of hypertension, truncal obesity, bone fracture, osteoporosis and an increased susceptibility for particular infectious diseases. These adverse pharmacological effects likely represent an exaggeration of the physiological response. For example, the aberrant induction of apoptosis in osteocytes and osteoblasts plays a part in glucocorticoid-induced osteoporosis, while low-dose corticosteroid therapy emerges as a most effective concept to prevent bone destruction during inflammatory diseases associated with bone loss such as rheumatoid arthritis and periodontal disease. The variability in the glucocorticoid response with respect to resistance, tissue-specificity and side-effects presents a challenge for researchers and clinicians and has prompted the design of selective, safer synthetic glucocorticoids that evade resistance. The physiological and pharmacological actions of glucocorticoids are predominantly mediated through the ubiquitously expressed glucocorticoid receptor (GR). To date, only one GR gene has been identified in all species examined. In order to ensure high specificity for the desired biological response of endogenously released hydrocortisone various levels of control and complex mechanisms of interactions of the GR with other nuclear receptors have emerged. These range from pre-receptor ligand metabolism and glucocorticoid signaling through the heterogeneous GR to the recruitment of cofactors to the GR receptor/ligand complex and its capability to form homo- or heterodimers for example with the mineralocorticoid receptor. The latter itself is the target of endogenous cortisol, which has a 10-30 fold higher affinity to the mineralocorticoid receptor then its classic endogenous agonist aldosterone. Furthermore, cortisol exceeds plasma levels of aldosterone by 100-1000 fold.

The molecular mechanisms that control cortisol actions have the potential to explain the etiology of disorders such as obesity and the variability in the glucocorticoid response.

Cortisol (the most abundant, endogenous glucocorticoid in man) is synthesized by the adrenal gland and transported in the blood predominantly bound (80%) to corticosteroid binding globulin (CBG). In addition to facilitating cortisol distribution, CBG has a role in tissue-specific cortisol release. Several lines of evidence indicate that cortisol delivery to precise microenvironments is accomplished through human-leukocyte-elastase-mediated CBG cleavage or membrane-bound CBG receptors, both of which stimulate the release of cortisol from CBG. CBG-free cortisol readily diffuses across cellular membranes to exert its effect.

Cortisol is crucial for maintaining physiological homeostasis, as exemplified by phenotypes associated with cortisol dysregulation. For example, tumors of the adrenal gland commonly produce excess cortisol, causing a pathological condition known as Cushing's syndrome, which is characterized by visceral obesity and metabolic disease (type-2 diabetes, insulin resistance, dyslipidemia and hypertension). The detrimental effect of cortisol excess is attributed to the induction of multiple metabolic and hemodynamic pathways in a variety of tissues, such as liver, kidney and adipocytes. Although similar features exist between Cushing's syndrome and the more common metabolic syndrome, metabolic syndrome seems to occur in the absence of systemic cortisol excess. This observation gave rise to the suggestion that intracellular cortisol excess, which is not detected by conventional methods, might contribute to the development of metabolic syndrome. Upon diffusion into the cytoplasm, the intracellular bioavailability of cortisol is controlled by a mechanism that has been termed 'prereceptor ligand metabolism'. 11-beta-hydroxysteroid dehydrogenase type 2 (11-beta-HSD2) catalyzes the conversion of cortisol to cortisone, the inactive glucocorticoid metabolite, whereas 11-beta-hydroxysteroid dehydrogenase type 1 (11-beta-HSD1) converts cortisone to cortisol. Thus, the relative levels of 11-beta-HSD enzymes are important factors in determining the intracellular concentration of cortisol. The major role of 11-beta-HSD2 is to prevent cortisol from gaining access to high-affinity mineralocorticoid receptors (MR) and, therefore, is predominantly expressed in the mineralocorticoid responsive cells of the kidney. By contrast, 11-beta-HSD1 ensures intracellular cortisol bioavailability in the glucocorticoid-responsive metabolic tissues of the liver, fat, the lung and the central nervous system, but also lymphoid tissues as well fibroblast in synovium and activated macrophages. The full range of 11-beta-HSD1 expressing tissues in human tissues has not been completely described. This cell type-specific expression pattern reflects selective activation of the 11-beta-HSD promoters and is important for fine-tuning glucocorticoid responses. Because cortisol has a crucial role in maintaining homeostasis, inappropriate tissue-specific alterations in 11-beta-HSD enzymes potentially might have detrimental effects on physiological processes. Indeed, recent studies provide initial data that indicate a role for 11-beta-HSD and intracellular cortisol excess in obesity and the development of metabolic disease. For example, 11-beta-HSD1-knockout mice are protected from high-fat-diet-induced pre-adipocyte differentiation and obesity, and parameters of cortisone-induced diabetes are improved in 11-beta-HSD1-knockdown mice. By contrast, transgenic mice that overexpress 11-beta-HSD1 specifically in the liver exhibit signs of metabolic disease, such as fatty liver, insulin resistance and hypertension, even in the absence of obesity. Furthermore, mice that overexpress 11-beta-HSD2 specifically in adipocytes are protected from high-fat diet-induced obesity. Clinical studies in humans have found elevated levels of 11-beta-HSD1 mRNA, decreased levels of 11-beta-HSD2 mRNA and increased cortisol levels in adipose tissue from obese subjects when compared to lean subjects. Moreover, there is an association between high levels of 11-beta-HSD1 mRNA and metabolic abnormalities in obese women. Together, these findings indicate that pre-receptor ligand metabolism regulates intracellular cortisol bioavailability and controls tissue-specific, endogenous glucocorticoid action. Because of the implications for the development of obesity and metabolic disease, these discoveries have prompted the design of 11-beta-HSD1 inhibitors for the treatment of metabolic syndrome.

Here it must be emphasized that the uniform reaction directions stipulated for 11-beta-HSD1 (only reductase *in vivo*) and 11-beta-HSD2 (only dehydrogenase) may at least with respect to 11-beta-HSD1 need some reconsideration. Enzymatic reductase activity of 11-beta-HSD1 activity is highly depended on the availability of its co-factor NADPH and the latter is influenced by a plethora of systems, not least endoplasmic stress and glucose availability. Furthermore, competition for the substrate binding pocket may additionally influence tissue-specific cortisol levels, in as much as 11-beta-HSD1 can also convert 7-keto-cholesterol and DHEA and some of its metabolites.

Role of MR in non-epithelial tissues: challenging the classical model of MR activation.

Although the classical model of MR activation and mode of action depicts most of the mineralocorticoid effects in epithelial target tissues, it fails to explain several observations of MR function in non-epithelial tissues such as the heart, the vasculature, macrophages, certain regions of the brain and human bone. 11-beta-HSD2 is expressed in sodium-transporting epithelial cells, where it can protect MR from glucocorticoid activation, but not in non-epithelial aldosterone-sensitive tissues, suggesting that other molecular mechanisms render specificity of MR for aldosterone or that the receptor might function as a glucocorticoid sensor in non-epithelial tissues.

MR and GR recognize the same conserved DNA sequences (glucocorticoid response element, GRE) in promoters of target genes, and MR-responsive genes can generally also be induced upon activation of GR. Most cells expressing MR also express GR, and a partial colocalization of the two receptors has been observed, for example in the nucleus of CA1 neurons in rat hippocampus. Coexpression of both receptors in combination with identical DNA recognition sites indicates a partial if not full overlap of MR- and GR-target genes. In addition, evidence for heterodimerization of the two receptors with distinct transactivation properties of the GR/MR heterodimer compared with the respective homodimers has been provided. Analysis of the dynamics of GR-MR complex formation in living cells by fluorescence resonance energy transfer (FRET) indicated that heterodimer formation occurs after the activation and nuclear translocation of each receptor. The close functional interactions and partially overlapping target genes of MR and GR can probably be better understood with respect to the evolutionary origin of MR. While the aldosterone and the aldosterone synthase genes are first reliably found in terrestrial vertebrates, the MR can be found in fish, suggesting that the MR is activated by a different principal ligand in fish or that it serves as a high-affinity receptor for cortisol. The latter hypothesis is supported by the abundant expression of MR in non-epithelial tissues in fish as well as in other species. Aldosterone synthase and aldosterone-mediated epithelial responses in higher vertebrates probably evolved later as an adaptation to the terrestrial environment, which differs from the aquatic environment with regard to fluid and electrolyte composition and abundance.

The higher affinity of MR for aldosterone and 11-beta-hydroxyglucocorticoids compared with GR and the fact that the circulating glucocorticoid concentration is several orders of magnitude greater than that of aldosterone suggests that the MR is permanently occupied by glucocorticoids in nonepithelial tissues lacking 11-beta-HSD2. Although glucocorticoid levels in the blood exhibit a diurnal rhythm with a nadir early in the morning, basal glucocorticoid levels are thought to be sufficiently high to always occupy the high-affinity MR, whereas the GR is only fully activated at peak glucocorticoid levels or during stress responses. It was demonstrated in rat hippocampal neurons that the MR is predominantly occupied in the nucleus, whereas nuclear translocation of GR occurs mainly during peak corticosterone levels. Importantly, the gate-keeping 11-beta-HSD2 is absent in hippocampus; instead, 11-beta-HSD1 is expressed. Although 11-beta-HSD1 catalyzes both the oxidation of 11-beta-hydroxyglucocorticoids and the reduction of 11-ketoglucocorticoids *in vitro*, it predominantly functions as a reductase *in vivo*. Experiments with cultured hippocampal cells demonstrated conversion of 11-dehydrocorticosterone to corticosterone, suggesting that 11-beta-HSD1 functions as a reductase in hippocampus and that the MR is occupied by corticosterone.

Several recent studies described considerable adverse effects of elevated aldosterone levels on the heart and kidney in various experimental models such as high salt/aldosterone, high salt/deoxycorticosterone, high salt/angiotensin II and stroke-prone spontaneously hypertensive rats, and significant improvements were observed upon application of MR blockers. Since an overactivation of the RAAS results in hypertension, it was initially thought that part of the detrimental effects of elevated mineralocorticoids might be a direct consequence of increased blood pressure on the vasculature.

However, rats infused peripherally with aldosterone and intracerebroventricularly with MR antagonist RU28318 had normal blood pressure but displayed adverse cardiac effects resembling those animals treated peripherally with aldosterone but receiving vehicle intra-cerebroventricularly. In addition, development of cardiac hypertrophy and fibrosis could be prevented by co-administration of the MR antagonists spironolactone or potassium canrenoate at doses that had no significant effect on blood pressure, suggesting that the adverse effects on the heart are caused by a direct mechanism rather than secondary hemodynamic effects. Furthermore, it was shown that left-ventricular hypertrophy is more common in patients with primary aldosteronism than in patients with other forms of hypertension, including essential hypertension, Cushing's syndrome and pheochromocytoma. Plasma aldosterone levels were also shown to correlate with decreased systemic arterial compliance in subjects with essential hypertension, independent of age and blood pressure. Increasing evidence indicates that MR antagonists are also beneficial in situations where aldosterone levels are normal or even low, suggesting MR activation by alternative ligands or ligand-independent activation. In clinical studies of patients with heart failure (and normal or low aldosterone levels) supplementation of standard therapy (ACE inhibitor, loop diuretic and digoxin) with the MR antagonists spironolactone or eplerenone decreased the post-myocardial infarction morbidity and mortality rate by about 30% and 15%, respectively. A recent clinical study with eplerenone in patients with general hypertension demonstrated reduced artery wall stiffness and an improved blood profile with respect to inflammatory cytokines and suggested that application of MR antagonists may be a superior alternative to the traditional treatment with beta-blockers such as atenolol. The mechanisms of MR activation in the vasculature and in the heart are still unclear. Vascular smooth muscle cells co-express MR and 11-beta-HSD2, and inhibition of 11-beta-HSD2 by the unselective inhibitor carbenoxolone in uninephrectomized rats receiving 0.9% NaCl solution to drink showed elevated systolic blood pressure and increased heart and kidney weight. The coronary vascular inflammatory response in carbenoxolone treated animals was comparable to that observed in aldosterone treated animals, suggesting that glucocorticoid-dependent MR activation in vascular smooth muscle cells plays a critical role in the development of impaired vascular function and organ damage.

In contrast to vascular smooth muscle cells, which may be considered as a physiological aldosterone target, cardiomyocytes express MR but not 11-beta-HSD2 and MR is occupied by glucocorticoids. It was demonstrated in rats that eplerenone but not steroid withdrawal can reverse mineralocorticoid induced cardiac fibrosis. The simultaneous infusion of corticosterone with aldosterone significantly attenuated aldosterone-induced cardiac fibrosis, indicating antagonistic effects of glucocorticoids on aldosterone-induced MR activation in the heart. One of the critical factors mediating adverse effects of aldosterone in the heart might be IL-18, which promotes myocardial hypertrophy and impairs cardiomyocyte contractility. Aldosterone was found to induce IL-18 expression by a mechanism involving endothelin-1 and angiotensin-II that seem to act through Rho/Rho-kinase and PPARs. Interestingly, transgenic mice overexpressing 11-beta-HSD2 in cardiomyocytes spontaneously developed dilated cardiomyopathy, fibrosis and heart failure, and died prematurely on a normal salt diet. The MR antagonist eplerenone ameliorated these effects, emphasizing the protective effect of physiological glucocorticoids occupying MR under basal conditions and preventing the adverse effects of aldosterone on cardiomyocytes if glucocorticoids are low. As suggested recently, in cardiomyocytes the MR may be activated by cortisol, dependent on changes in the intracellular redox potential. Thus, MR antagonists may act through different mechanisms in cardiomyocytes and vascular cells and prevent cardiovascular damage. MR blockade was recently reported to reverse obesity related changes in adipose tissue and in the heart. Treatment with eplerenone decreased the expression of proinflammatory and prothrombotic factors in adipose tissue and enhanced the expression of adiponectin in heart and adipose tissue of obese and diabetic mice. Undifferentiated preadipocytes incubated in the presence of low concentrations of aldosterone showed increased expression of TNF-alpha and monocyte chemoattractant protein-1 and decreased levels of PPAR-gamma and adiponectin. Furthermore, MR antagonists may exert beneficial effects by modulating the response of macrophages to inflammatory stimuli. MR is expressed in macrophages in the presence of the glucocorticoid activating 11-beta-HSD1. Whether glucocorticoids antagonize MR activation in macrophages and the molecular mechanism underlying MR activation remain to be elucidated. Nevertheless, observations from animal experiments indicated that aldosterone promotes the production of proinflammatory cytokines, vascular macrophage infiltration and atherosclerosis. Caprio et al. recently reported the expression of MR and 11-beta-HSD2 in human vascular endothelial cells. They showed that aldosterone stimulates the expression of the proatherogenic leukocyte-endothelial cell adhesion molecule ICAM-1 as well as leukocyte-endothelial cell adhesion. These recent observations indicate important functions of MR in the regulation of inflammation.

Another open question regarding corticosteroid receptor function remains the exact mechanism of GR activation in cells expressing high levels of 11-beta-HSD2, including the classical mineralocorticoid targets in the kidney (cortical collecting ducts and distal tubules) and colon (distal colon). These cell types co-express 11-beta-HSD2, MR and GR, whereby the number of GR complexes exceeds that of MR by at least one order of magnitude. In these tissues 11-beta-HSD2 serves to protect the high-affinity MR by efficiently inactivating glucocorticoids. The function of the lower affinity GR in these cells is therefore uncertain, since it is not clear how GR can be activated in the presence of 11-beta-HSD-2, which catabolises endogenous cortisol, but not most synthetic corticosteroids currently used in the clinical setting. Apparently the interplay between the MR and GR within a cell may not necessarily require full receptor occupation, possibly exploiting that GR and MR can assemble to heterodimers. The physiological conditions under which this phenomenon takes place are not characterized yet, but its physiological relevance has been documented in several studies.

In summary, if the endogenous cortisol level is manipulated, this will ultimately lead to a shift in the balance between GR and MR induced effects on gene transcription. Here it must be emphasized that the relative contribution of either receptor is highly depending on the relevant tissue and the collection of available cofactors for recruitment. Furthermore, depending on the ligand (aldosterone or cortisol) that occupies the binding pocket, a different set of coregulators might be attracted to form a complex which results in a level of complexity that is best described as stochastic. This phenomenon may partially serve to explain, why the presence of pathologic conditions that are attributable to excessive cortisol generation in one tissue (like obesity) may coincide with pathologies associated with a lack of appropriate regeneration of cortisol (like rheumatoid arthritis). Thus, it is currently impossible to delineate the outcome of a manipulation of endogenous cortisol levels on the distinct actions of MR occupied by cortisol, but it appears that this interactions in the magnitude of instances results in detrimental induction or perpetuation of inflammatory reaction in vascular tissues, bone or lung, all of which express both, GR and MR.

From a pragmatic therapeutic standpoint, it has become clear that occupancy of MR by cortisol contributes to vascular inflammation, worsens pathologies like ischemic stroke and other undesired effects, previously attributed to the occupancy of the glucocorticoid receptor in a given tissue or cell type; of note, this appears to be of particular relevance in tissues that differ in their expression pattern from one species to the other. For example, mature human osteoclast do not express GR-alpha, but GR-beta and MR.

Blocking 11-beta-HSD1 has become a most attractive avenue to treat conditions associated with increased tissue-specific generation from cortisol, such as diabetes type 2. As a result, lowering endogenous cortisol by an 11-beta-HSD inhibitor would still allow occupancy of the high affinity receptor MR with cortisol or aldosterone in conditions associated with increased aldosterone. While lowering cortisol in some tissue would reduce glucocorticoid-induced insulin resistance, it would ultimately result in increased vascular inflammation and stiffening of vessels or other tissues like the lung or induction/perpetuation of bone destruction. In this context it appears seemingly paradox, that blocking the MR also ameliorates pathologies summarised as metabolic diseases, in particular diabetes type II, cardiovascular diseases as well as other conditions related to increase angiogenesis, like diabetic retinopathy. Over all it appears that the complex interplay between the GR, MR and their ligands and coregulators is more complex then previously anticipated, which results in seemingly counterintuitive results as discussed here.

### Summary of the invention

In order to prevent undesired effects induced by dominant signalling of the occupied MR under low cortisol conditions (through blockade of 11-beta-HSD-1 reductase by an appropriate small molecule inhibitor) it has been surprisingly found that addition of an MR-antagonist (and/or inhibitor) (such as eplerenone) in a combination treatment ablates the induction of effects like oxidative stress.

Unexpectedly, this combination treatment did not only result in amelioration of diabetes-related pathologies, but also reduced the over-all inflammatory response in a non-additive but exponential manner.

Thus, according to the present invention, a combination treatment using a steroid-dehydrogenase-reductase inhibitor combined with a mineralocorticoid receptor antagonist overcomes the problem that manipulating cortisol levels by a single compound therapy may induce other pathologies or side effects, while the original condition that required treatment is ameliorated.

The present invention provides a therapy using a combination of
a) a steroid-dehydrogenase-reductase inhibitor (especially an inhibitor of 11-beta-HSD reductase) and
b) a mineralocorticoid receptor antagonist (and/or inhibitor).

The present invention further provides a pharmaceutical composition comprising:
a) a steroid-dehydrogenase-reductase inhibitor (especially an inhibitor of 11-beta-HSD reductase) and
b) a mineralocorticoid receptor antagonist (and/or inhibitor).

Further, the present invention provides a kit of parts comprising:
a) a steroid-dehydrogenase-reductase inhibitor (especially an inhibitor of 11-beta-HSD1 or 11-beta-HSD2) and
b) a mineralocorticoid receptor antagonist.

Further, the present invention provides a kit of parts comprising:
a) a pharmaceutical composition comprising a steroid-dehydrogenase-reductase inhibitor (especially an inhibitor of 11-beta-HSD1 or 11-beta-HSD2) and
b) a pharmaceutical composition comprising a mineralocorticoid receptor antagonist.

Within the context of the present invention, HSD refers to "hydroxysteroid dehydrogenase reductase" and MR refers to "mineralocorticoid receptor".

Preferably, the steroid dehydrogenase reductase inhibitor and the mineralocorticoid receptor antagonist are different compounds.

According to the present invention, in a disease condition where 11-beta-HSDs (type 1 and/or type 2) alone or in combination in a given tissue are causative for an undesired catabolism of endogenous cortisol and resulting in exaggerated inflammation, blocking of either enzyme or both will lead to increased endogenous cortisol levels, resulting in increased anti-inflammatory actions mediated via the GR, will also result in undesired MR activation and hence side-effects.

This effect is particularly detrimental in tissues where MR activation is normally prevented by 11-beta-HSD2. Under these conditions, addition of an MR-antagonist in combination with an 11-beta-HSD inhibitor prevented the activation of an inflammatory cascade as observed in vascular pathologies of the eye, brain and heart. 11-beta-HSD activity may be mediated via 11-beta-HSD1 under particular conditions and/or 11-beta-HSD2.

With respect to a protection against bone loss induced by inflammation or immune responses, the activation of osteoclasts by cortisol acting on the MR was also prevented and contributed to the overall resolution from disease.

According to the present invention, the MR-antagonist (and/or inhibitor) may preferably be selected from but is not limited to the following compounds: 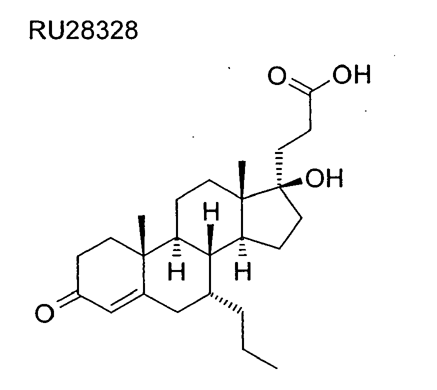 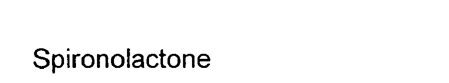 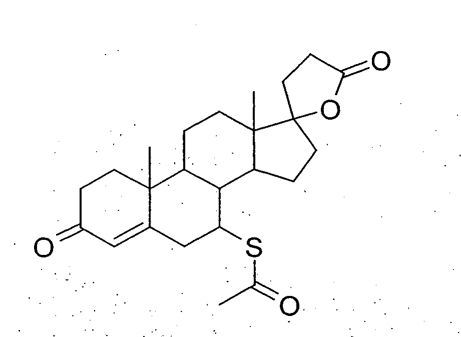 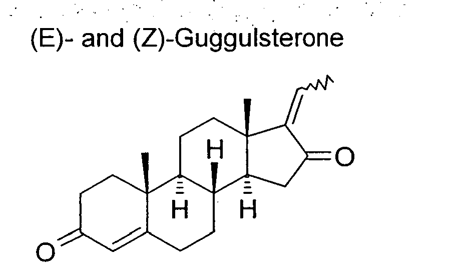 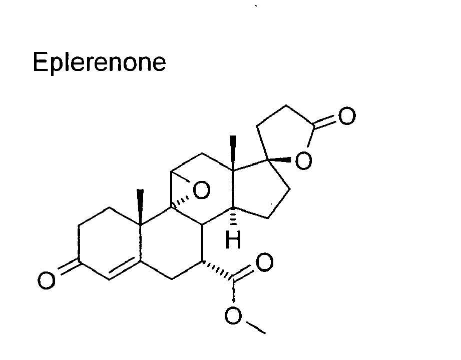 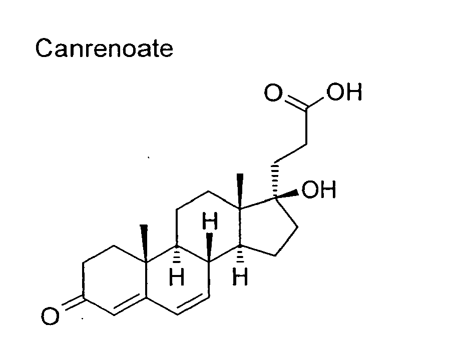 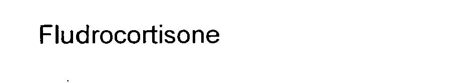 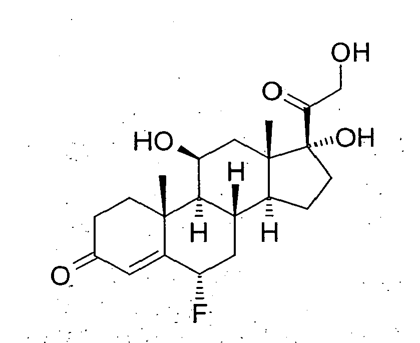 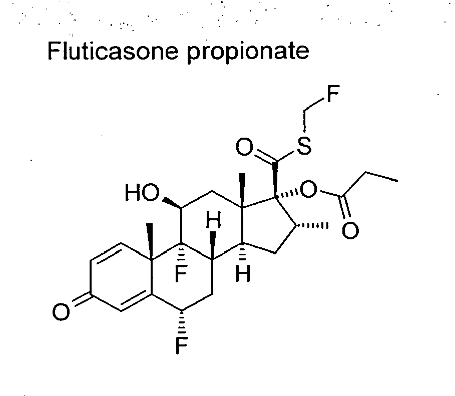 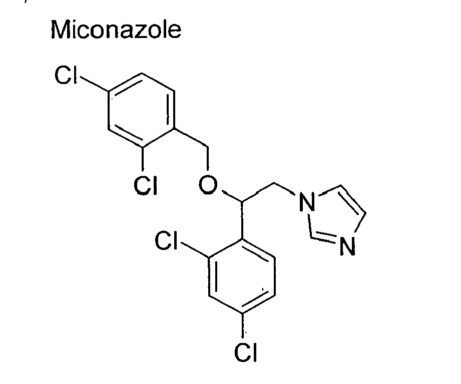 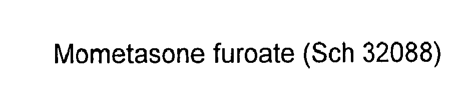 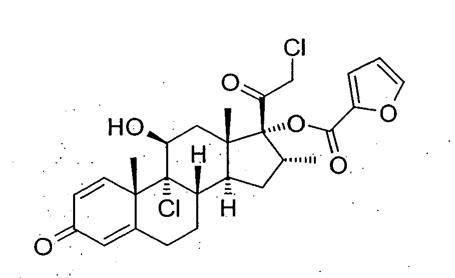 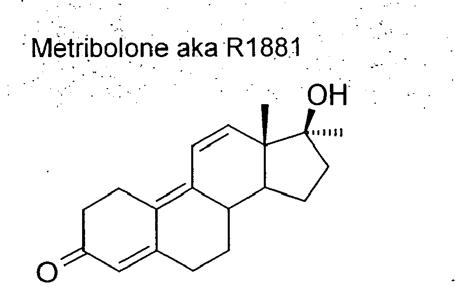 or a compound selected from 2-(3-chloro-4-cyanophenyl)-3-(4-fluorophenyl)-3,3a,4,5-tetrahydro-2H-benzo[g]indazole-7-carboxylic acid, 3-(4-fluorophenyl)-7-hydroxy-3,3a,4,5-tetrahydrobenzo[g]indazol-2-yl)-2-methylbenzonitrile, 3-(4-fluorophenyl)-7-hydroxy-3,3a,4,5-tetrahydrobenzo[g]indazol-2-yl)-2-trifluoromethyl-benzonitrile, 2-chloro-4-(3-(4-fluorophenyl)-7-hydroxy-3,3a,4,5-tetrahydrobenzo[g]indazol-2-yl)benzonitrile, 2-(3-chloro-4-cyanophenyl)-3-(4-fluorophenyl)-N-(2-(methylsulfonyl)ethyl)-3,3a,4,5-tetrahydro-2H-benzo[g]indazole-7-carboxamide, 2-(3-chloro-4-cyanophenyl)-3-cyclopentyl-3,3a,4,5-tetrahydro-2H-benzo[g]indazole-7-carboxylic acid, 2-(4-cyanophenyl)-3-cyclopentyl-3,3a,4,5-tetrahydro-2H-benzo[g]indazole-7-carboxylic acid, 2-(4-cyano-3-methylphenyl)-3-cyclopentyl-3,3a,4,5-tetrahydro-2H-benzo[g]indazole-7-carboxylic acid, 2-(5-cyano-6-methylpyridin-2-yl)-3-cyclopentyl-3,3a,4,5-tetrahydro-2H-benzo[g]indazole-7-carboxylic acid, 2-(4-cyano-3-methoxyphenyl)-3-cyclopentyl-3,3a,4,5-tetrahydro-2H-benzo[g]indazole-7-carboxylic acid, N-(2-(3-chloro-4-cyanophenyl)-3-cyclopentyl-3,3a,4,5-tetrahydro-2H-benzp[g]indazol-7-yl)-acetamide, methyl 2-(3-chloro-4-cyanophenyl)-3-cyclopentyl-3,3a,4,5-tetrahydro-2H-benzo[g]indazole-7-carboxylate, 2-(3-chloro-4-cyanophenyl)-3-cyclopentyl-3a-methyl-3,3a,4,5-tetrahydro-2H-benzo[g]indazole-7-carboxylic acid, 2-(3-chloro-4-cyanophenyl)-3cyclopentyl-N-(2-(methylsulfonyl)ethyl)-3,3a,4,5-tetrahydro-2H-benzo[g]indazole-7-carboxamide, 2-(3-chloro-4-cyanophenyl)-3-cyclopentyl-N-(2-hydroxyethyl)-3,3a,4,5-tetrahydro-2H-benzo[g]indazole-7-carboxamide, 2-(4-cyano-3-methylphenyl)-3-cyclopentyl-2,3,3a,4-tetrahydro-chromeno[4:3c]pyrazole-7-carboxylic acid, 2-(3-chloro-4-cyanophenyl)-3-cyclobutyl-3,3a,4,5-tetrahydro-2H-benzo[g]indazole-7-carboxylic acid, 2-(3-chloro-4-cyanophenyl)-3-cyclopentenyl-3,3a,4,5-tetrahydro-2H-benzo[g]indazole-7-carboxylic acid, 2-(4-cyano-3-methylphenyl)-3-cyclopentenyl-3,3a,4,5-tetrahydro-2H-benzo[g]indazole-7-carboxylic acid, 2-(3-chloro-4-cyanophenyl)-3-(5-methyl-2-furyl)-3,3a,4,5-tetrahydro-2H-benzo[g]indazole-7-carboxylic acid, 2-(3-chloro-4-cyanophenyl)-3-(3-furyl)-3,3a,4,5-tetrahydro-2H-benzo[g]indazole-7-carboxylic acid, 2-(3-chloro-4-cyanophenyl)-3-(5-methyl-2-furyl)-N-[2-(methylsulfonyl)ethyl]-3,3a,4,5-tetrahydro-2H-benzo[g]indazole-7-carboxamide, 2-(3-chloro-4-cyanophenyl)-N-[2-(methylsulfonyl)ethyl]-3-(2-methyl-1,3-thiazol-5-yl)-3,3a,4,5-tetrahydro-2H-benzo[g]indazole-7-carboxamide, 2-(3-chloro-4-cyanophenyl)-3-cyclopentyl-2,3,3a,4-tetrahydrochromeno[4:3c]pyrazole-7-carboxylic acid, 2-[4-cyano-3-(trifluorornethyl)phenyl]-3-cyclopentyl-2,3,3a,4-tetrahydro-chromeno[4,3c]pyrazole-7-carboxylic acid, 2-(4-cyano-3-methylphenyl)-3-cyclopentenyl-2,3,3a,4-tetrahydrochromeno[4:3c]pyrazole-7-carboxylic acid, and 2-(3-chloro-4-cyanophenyl)-3-cyclopentenyl-2,3,3a,4-tetrahydrochromeno[4:3c]pyrazole-7-carboxylic acid, 2-(3-chloro-4-cyanophenyl)-3-cyclopentyl-3,3a,4,5-tetrahydro-2H-benzo[g]indazole-7-carboxylic acid, 2-(4-cyano-3-methylphenyl)-3-cyclopentyl-3,3a,4,5-tetrahydro-2H-benzo[g]indazole-7-carboxylic acid, and 2-(4-cyano-3-methoxyphenyl)-3-cyclopentyl-3,3a,4,5-tetrahydro-2H-benzo[g]indazole-7-carboxylic acid.

According to the present invention, the inhibitors of 11-beta-HSD reductase may preferably be selected from but not being limited to the compounds described by SP Webster and TD Pallin in Expert Opin. Ther. Patents (2007) 17(12): 1407-1422 or KA Hughes, SP Webster, BR Walker Expert Opin. Invest. Drugs (2008) 17(4):481-496 which are incorporated by reference.

According to the present invention, the inhibitors of 11-beta-HSD reductase may especially preferred be selected from but are not limited to the following compounds: 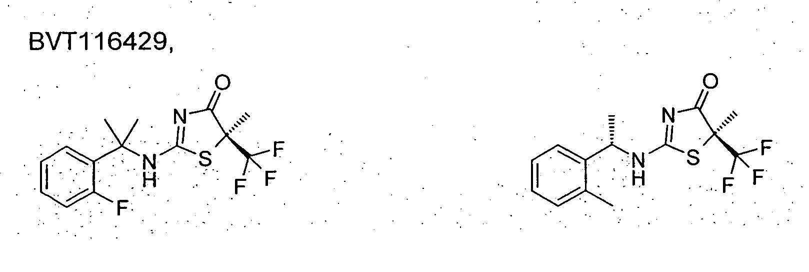 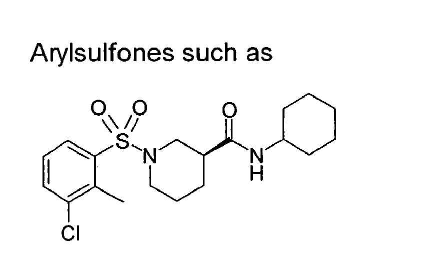 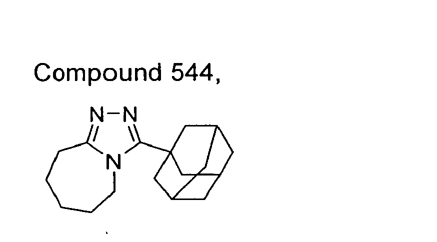 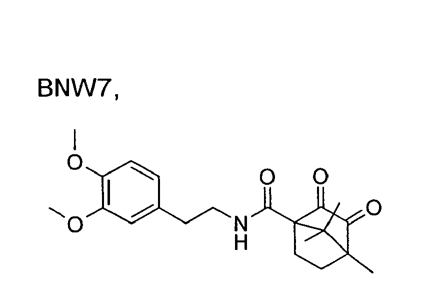 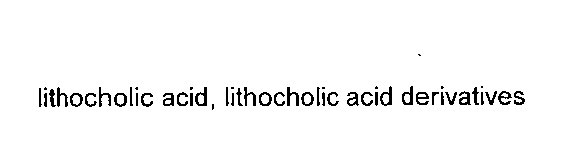 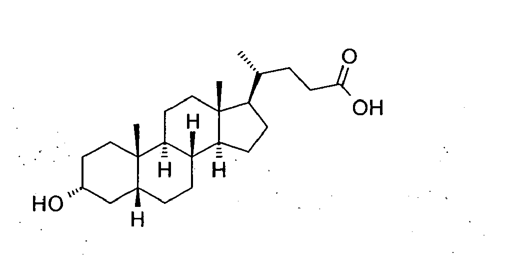 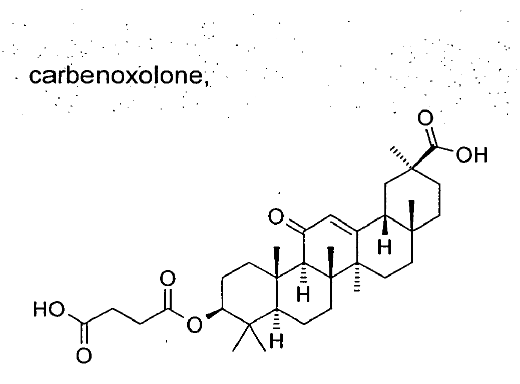 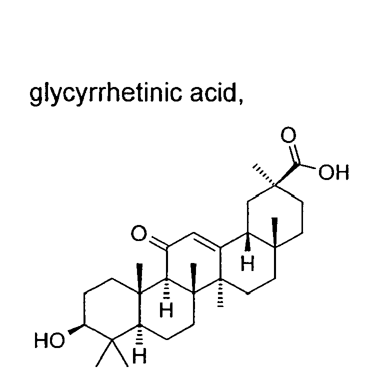 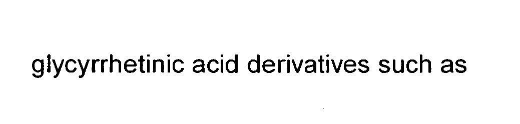 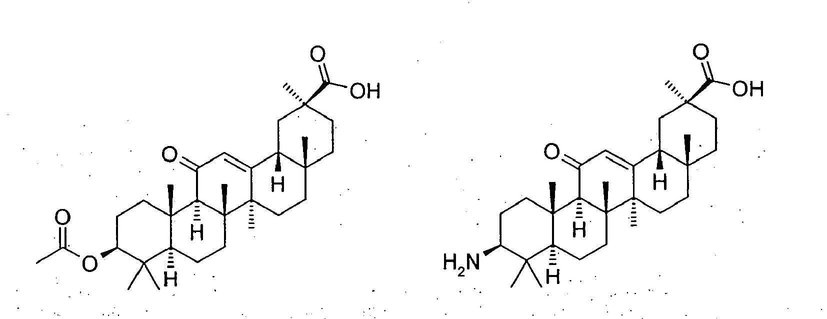 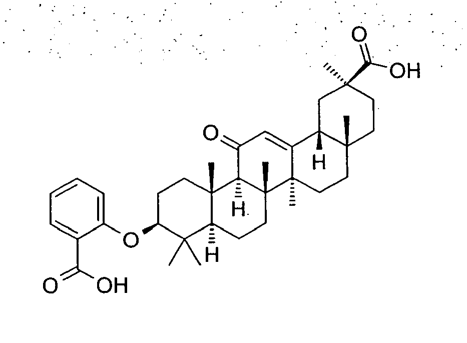 or a compound selected from 5-chloro-1-(4-tert-butylphenylsulfonyl)-2,3-dihydroindole, 3-(R)-1-(4-tert-butylphenylsulfonyl)-3-imidazol-1-yl)-pyrrolidine, 3-(R)-1-(4-tert-butylphenylsulfonyl)-3-methoxypyrrolid ine, 2-(S)-2-methyl-1-(4-tert-butylphenylsulfonyl)-morpholine, syn-2,6-dimethyl-1-(4-(2,2,2-trifluoro-1-hydroxy-1-methylethyl)phenyl-sulfonyl)-piperidine, 1-(4-(2,2,2-trifluoro-1-hydroxy-1-methylethyl)phenylsulfonyl)-homopiperidine, 2-(R)-2-methyl-1-(4-(2,2,2-trifluoro-1-hydroxy-1-methylethyl)phenyl-sulfonyl)-piperidine, 2-(S)-2-methyl-1-(4-(2,2,2-trifluoro-1-hydroxy-1-methylethyl)phenyl-sulfonyl)-piperidine, 2-ethyl-1-(4-(2,2,2-trifluoro-1-hydroxy-1-methylethyl)phenylsulfonyl)-piperidine, 1-(4-(2,2,2-trifluoro-1-hydroxy-1-methylethyl)phenylsulfonyl)-piperidine, 2-(4-(2,2,2-trifluoro-1-hydroxy-1-methylethyl)phenylsulfonyl)-1,2,3,4-tetrahydroisoquinoline, 2-(S)-2-(pyridin-3-yl)-1-(4-(2,2,2-trifluoro-1-hydroxy-1-methylethyl)-phenylsulfonyl)-piperidine, 1-(4-(2,2,2-trifluoro-1-hydroxy-1-methylethyl)phenylsulfonyl)-1,2,3,4-tetrahydroquinoline, 1-(4-(2,2,2-trifluoro-1-hydroxy-1-methylethyl)phenylsulfonyl)-heptamethyleneimine, 3-fluoro-1-(4-(2,2,2-trifluoro-1-hydroxy-1-methylethyl)phenylsulfonyl)-piperidine, 1-(4-(2,2,2-trifluoro-1-hydroxy-1-methylethyl)phenylsulfonyl)-2-(2-imidazol-1-yl-ethyl)piperidine, 2-(2-pyrazol-1-yl-ethyl)-1-(4-(2,2,2-trifluoro-1-hydroxy-1-methylethyl)-phenylsulfonyl)-piperidine, 3-(R)-3-(piperidin-1-yl)-1-(4-(2,2,2-trifluoro-1-hydroxy-1-methylethyl)-phenylsulfonyl)-pyrrolidine and 2-(2-hydroxyethyl)-1-(4-(2,2,2-trifluoro-1-hydroxy-1-methylethyl)-phenylsulfonyl)-piperidine, ((1S)-2-{[1-(phenylthio)cyclopropyl]carbonyl}-1,2,3,4-tetrahydroisoquinolin-1-yl)methanol; 2-{[1-(phenylthio)cyclopropyl]carbonyl}-1,2,3,4-tetrahydroisoquinoline, 6-{[1-(phenylthio)cyclopropyl]carbonyl)}4,5,6,7-tetrahydrothieno[2,3-c]pyridine, 3-phenyl-1-{[1-(phenylthio)cyclopropyl]carbonyl}piperidine, 1'-{[1-(phenylthio)cyclopropyl]carbonyl}-1,3-dihydrospiro[indene-2,4'-piperidine]; 2-methyl-1-phenyl-4-{[1-(phenylthio)cyclopropyl]carbonyl}piperazine, 2-{[1-(phenylthio)cyclopropyl]carbonyl}-2,3,3a,4,5,9b-hexahydro-1H-benzo[e]isoindole, 3-(3-fluorophenyl)-1-{[1-(phenylthio)cyclopropyl]carbonyl}pyrrolidine, 1'-{[1-(phenylthio)cyclopropyl]carbonyl}-3H-spiro[2-benzofuran-1,3'-pyrrolidin]-3-one, ((3S)-2-{[1-(Phenylthio)cyclopropyl]carbonyl}-1,2,3,4-tetrahydroisoquinolin-3-yl)methanol, 2-(4-(Hydroxymethyl)-1-{[1-(phenylthio)cyclopropyl]carbonyl}pyrrolidin-3-yl)phenol, 2-{[1-(Phenylthio)cyclopropyl]carbonyl}-1,2,3,3a,4,9b-hexahydrochromeno[3,4-c]pyrrole, 1'-({1-[(4-Chlorophenyl)thio]cyclopropyl}carbonyl)-3H-spiro[2-benzofuran-1,3'-pyrrolidin]-3-one, 1-{[1-(Cyclohexylsulfonyl)cyclopropyl]carbonyl}pyrrolidine, 4-(1-{[1-(Cyclohexylsulfonyl)cyclopropyl]carbonyl}pyrrolidin-3-yl)pyridine, 4-[1-({1-[(4-Chlorophenyl)thio]cyclopropyl}carbonyl)pyrrolidin-3-yl]pyridine, 1-({1-[(4-Chlorophenyl)thio]cyclopropyl}carbonyl)-3-(3-fluorophenyl)pyrrolidine, 3-(4-Chlorophenyl)-1-({1-[(4-chlorophenyl)thio]cyclopropyl}carbonyl)pyrrolidine, 2-[1-({1-[(4-Chlorophenyl)thio]cyclopropyl}carbonyl)pyrrolidin-3-yl]pyridine, 1'-({1-[(3,5-Dichlorophenyl)thio]cyclopropyl}carbonyl)-3H-spiro[2-benzofuran-1,3'-pyrrolidin]-3-one, 1'-({1-[(3-Chloro-4-fluorophenyl)thio]cyclopropyl}carbonyl)-3H-spiro[2-benzofuran-1,3'-pyrrolidin]-3-one, 1'-({1-[(2,6-Dichlorophenyl)thio]cyclopropyl}carbonyl)-3H-spiro[2-benzofuran-1,3'-pyrrolidin]-3-one, 1'-({1-[(4-Fluorophenyl)thio]cyclopropyl}carbonyl)-3H-spiro[2-benzofuran-1,3'-pyrrolidin]-3-one, 1'-({1-[(4'-Fluorobiphenyl-4-yl)thio]cyclopropyl}carbonyl)-3H-spiro[2-benzofuran-1,3'-pyrrolidin]-3-one, 1'-({1-[(3,4-Dichlorophenyl)thio]cyclopropyl}carbonyl)-3H-spiro[2-benzofuran-1,3'-pyrrolidin]-3-one, 1'-[(1-{[3-(Trifluoromethyl)phenyl]thio}cyclopropyl)carbonyl]-3H-spiro[2-benzofuran-1,3'-pyrrolidin]-3-one, 1'-[(1-{[4-(Trifluoromethoxy)phenyl]thio}cyclopropyl)carbonyl]-3H-spiro[2-benzofuran-1,3'-pyrrolidin]-3-one, 1'-({1-[(4-Chlorophenyl)thio]cyclopropyl}carbonyl)-3 H-spiro[2-benzofuran-1,3'-pyrrolidine], 1'-({1-[(4'-Fluorobiphenyl-4-yl)thio]cyclopropyl}carbonyl)-3H-spiro[2-benzofuran-1,3'-pyrrolidine], 1-{[1-(Cyclohexylsulfonyl)cyclopropyl]carbonyl}-3-phenylpiperazine, 1-({1-[(4-Chloroohenyl)thio]cyclopropyl}carbonyl)-3-phenylpiperazine, 6-{[1-(Phenylthio)cyclopropyl]carbonyl}-3a,4,5,6,7,7a-hexahydrothieno[2,3-c]pyridine, (4aR,8aS)-2-({1-[(4-Chlorophenyl)thio]cyclopropyl}carbonyl)decahydroisoquinoline, 1-({1-[(4-Chlorophenyl)thio]cyclopropyl}carbonyl)decahydroquinoline, 3-Chloro-2-methyl-N-(2-methyl-pyrimidin-4-yl)-benzenesulfonamide, 3-Chloro-N-(2-cyclopropyl-pyrimidin-4-yl)-2-methyl-benzenesulfonamide, N-(2-Cyclopropyl-pyrimidin-4-yl)-2,5-difluoro-benzenesulfonamide, Naphthalene-2-sulfonic acid (2-cyclopropyl-pyrimidin-4-yl)-amide, Biphenyl-4-sulfonic acid (2-cyclopropyl-pyrimidin-4-yl)-amide, Quinoline-8-sulfonic acid (2-cyclopropyl-pyrimidin-4-yl)-amide, N-(2-Cyclopropyl-pyrimidin-4-yl)-benzenesulfonamide, N-(2-Cyclopropyl-pyrimidin-4-yl)-5-fluoro-2-methyl-benzenesulfonamide, N-(2-Cyclopropyl-pyrimidin-4-yl)-3-methoxy-benzenesulfonamide, N-(2-Cyclopropyl-pyrimidin-4-yl)-2-methoxy-5-methyl-benzenesulfonamide, 3-Chloro-N-(2-cyclopropyl-pyrimidin-4-yl)-4-methoxy-benzenesulfonamide, 5-Chloro-N-(2-cyclopropyl-pyrimidin-4-yl)-2-methoxy-benzenesulfonamide, 5-Fluoro-N-(2-isopropyl-pyrimidin-4-yl )-2-methyl-benzenesulfonamide, 3,4-Dichloro-N-(2-isopropyl-pyrimidin-4-yl)-benzenesulfonamide, N-(2-Isopropyl-pyrimidin-4-yl)-4-(1.3-oxazol-5-yl)-benzenesulfonamide, 2,4-Dichloro-N-(2-isopropyl-pyrimidin-4-yl)-6-methyl-benzenesulfonamide, 2,3-Dichloro-N-(2-isopropyl-pyrimidin-4-yl)-benzenesulfonamide, 4,5-Dichloro-thiophene-2-sulfonic acid (2-isopropyl-pyrimidin-4-yl)-amide, 5-Pyridin-2-yl-thiophene-2-sulfonic acid (2-isopropyl-pyrimidin-4-yl)-amide, 3-Chloro-N-(2-isopropyl-pyrimidin-4-yl)-2-methyl-benzenesulfonamide, N-(2-Isopropyl-pyrimidin-4-yl)-3-trifluoromethyl-benzenesulfonamide, N-(2-Isopropyl-pyrimidin-4-yl)-2-trifluoromethyl-benzenesulfonamide, 5-Chloro-thiophene-2-sulfonic acid (2-isopropyl-pyrimidin-4-yl)-amide, N-(2Isopropyl-pyrimidin-4-yl)-4-trifluoromethyl-benzenesulfonamide, Piperidine-1 -sulfonic acid (2-isopropyl-pyrimidin-4-yl)-amide, Naphthalene-2-sulfonic acid (2-isopropyl-pyrimidin-4-yl)-amide, Biphenyl-4-sulfonic acid (2-isopropyl-pyrimidin-4-yl)-amide, 2,5-Difluoro-N-(2-isopropyl-pyrimidin-4-yl)-benzenesulfonamide, N-(2-Isopropyl-pyrimidin-4-yl )-3,4-dimethoxy-benzenesuifonamide, N-(2-tert-Butyl-pyrimidin-4-yl )-3,4-dichloro-benzenesulfonamide, N-(2-tert-Butyl-pyrimidin-4-yl)-5-fluoro-2-methyl-benzenesulfonamide, Naphthalene-2-sulfonic acid (2-tert-butyl-pyrimidin-4-yl)-amide, N-(2-tert-Butyl-pyrimidin-4-yl)-2,5-difluoro-benzenesulfonamide, N-(2-tert-Butyl-pyrimidin-4-yl)-4-(1.3-oxazol-5-yl)-benzenesulfonamide, 3-Chloro-N-(2-ethyl-pyrimidin-4-yl)-2-methyl-benzenesulfonamide, 2,4-Dichloro-N-(2-ethyl-pyrimidin-4-yl)-6-methyl-benzenesulfonamide, 4-Chloro-N-(2-ethyl-pyrimidin-4-yl )-2,5-dimethyl-benzenesulfonamide, 3-Chloro-N-(2-cyclobutyl-pyrimidin-4-yl)-2-methyl-benzenesulfonamide, Naphthalene-2-sulfonic acid (2-cyclobutyl-pyrimidin-4-yl)-amide, 5-Pyridin-2-yl-thiophene-2-sulfonic acid (2-cyclobutyl-pyrimidin-4-yl)-amide, 2,4-Dichloro-N-(2-cyclobutyl-pyrimid in-4-yl)-6-methyl-benzenesulfonamide, 3,4-Dichloro-N-(2-methoxymethyl-pyrimidin-4-yl)-benzenesulfonamide, 3-Chloro-N-(2-cyclopropylmethoxymethyl-pyrimidin-4-yl)-2-methyl-benzenesulfonamide, 3-Chloro-2-methyl-N-(2-morpholin-4-ylmethyl-pyrimidin-4-yl)-benzenesulfonamide, Naphthalene-2-sulfonic acid (2,6-dimethyl-pyrimidin-4-yl)-amide, 3-Chloro-N-(2-cyclopropyl-pyrimidin-4-yl)-2-N-dimethyl-benzenesulfonamide, 3,4-Dichloro-N-(2-isopropyl-pyrimidin-4-yl)-N-methyl-benzenesulfonamide, 3-Chloro-2-methyl-N-(2-phenyl-pyrimidin-4-yl)-benzenesulfonamide, and 3-Chloro-2-methyl-N-[2-(2-methyl-thiazol-4-yl)-pyrimidin-4-yl]-benzenesulfonamide.

Especially preferred is a combination of eplerenone or spironolactone with carbenoxolone, glycyrrhetinic acid, glycyrrhetinic acid derivatives (such as the ones shown above) or lithocholic acid or lithocholic acid derivatives.

The therapeutic use of the compounds described herein, their pharmacologically acceptable salts or solvates and hydrates and also formulations and pharmaceutical compositions also lie within the scope of the present invention, especially, the use of the compounds described herein, their pharmacologically acceptable salts or solvates and hydrates and the pharmaceutical compositions and also formulations for the treatment and/or prevention of conditions associated with unadequate aldosterone or cortisol; undesired effects of increased glucocorticoids associated with occupation of MR by cortisol as observed in cardiovascular diseases, stroke, vascular disease of the eye, hearth and lung, osteoporosis, rheumatoid arthritis, periodontitis, inflammatory bowel disease; colitis ulcerosa, asthma, chronic obstructive pulmonary disease and inflammatory skin diseases, and cancer related to increased angionesis such as: colon cancer, osteosarcoma, non small cell bronchial carcinoma, breast cancer, renal cell carcinoma, urinary bladder carcinoma, acute lymphoblastic leukaemia, endometriosis; conditions associated with occupation of MR either by aldosterone or cortisol; conditions associated with MR activation such as cardiovascular diseases, stroke, pathologic angiogenesis, metabolic syndrome; bone destruction in immune- and/or inflammation induced bone loss due to an underlying chronic inflammatory condition, such as osteoporosis, diabetes, colitis ulcerosa.

A further aspect of the present invention relates to the use of a combination of a MR-antagonist with a corticosteroid treatment for inflammatory diseases associated with decreased bone formation and/or increased bone destruction; in particular corticosteroid therapies in rheumatoid arthritis, periodontal diseases, asthma, inflammatory skin diseases,

Further, the present invention relates to the preparation of medicaments for the prevention and/or treatment of the diseases and/or conditions described above.

Packaged pharmaceutical preparations are also provided, comprising a pharmaceutical composition according to the present invention in a container and instructions for using the composition to treat a patient suffering from one of these diseases.

The pharmaceutically compositions according to the present invention comprise preferably at least one compound (a) and one compound (b) described herein and, optionally, carrier substances and/or adjuvants. Further, these pharmaceutically compositions may comprise other pharmaceutically active ingredients.

Examples of pharmacologically acceptable salts of the compounds described herein are salts of physiologically acceptable mineral acids, such as hydrochloric acid, sulphuric acid and phosphoric acid, or salts of organic acids, such as methanesulphonic acid, p-toluenesulphonic acid, lactic acid, acetic acid, trifluoroacetic acid, citric acid, succinic acid, fumaric acid, maleic acid and salicylic acid. Further examples of pharmacologically acceptable salts of the compounds described herein are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. The compounds described herein may be solvated, especially hydrated. The hydration may take place, for example, during the preparation process or as a consequence of the hygroscopic nature of the initially anhydrous compounds. When the compounds described herein comprise asymmetric C-atoms, they may be present either in the form of achiral compounds, diastereoisomeric mixtures, mixtures of enantiomers or in the form of optically pure compounds.

The present invention relates also to the use of those compounds in the preparation of medicaments. In general, the compounds and pharmaceutical compositions are administered either individually, or in combination with any other desired therapeutic agent, using the known and acceptable methods. Such therapeutically useful agents may be administered, for example, by one of the following routes: orally, for example in the form of dragées, coated tablets, pills, semi-solid substances, soft or hard capsules, solutions, emulsions or suspensions; parenterally, for example in the form of an injectable solution; rectally in the form of suppositories; by inhalation, for example in the form of a powder formulation or a spray; transdermally or intranasally. For the preparation of such tablets, pills, semi-solid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

For the prevention and/or treatment of the condition and/or diseases described above, the dose of the biologically active compounds according to the invention may vary within wide limits and may be adjusted to individual requirements. Generally, a dose of from 1 mg to 4000 mg per day is suitable, a preferred dose being from 5 to 3000 mg per day. In suitable cases, the dose may also be below or above the stated values. The daily dose may be administered as a single dose or in a plurality of doses. A typical individual dose contains approximately 1 mg, 5 mg, 10 mg, 20 mg, 50 mg, 100 mg, 250 mg, 500 mg, 1 g or 2 g of each active ingredient.

The ratio of the steroid-dehydrogenase-reductase inhibitor (a) and the mineralocorticoid receptor antagonist (and/or inhibitor) (b) may vary within wide limits and may be adjusted to individual requirements. A typical ratio (a) : (b) may e.g. range from 1 : 99 to 99 : 1; 10 : 90 to 90 : 10; 20 : 80 to 80 : 20; 30 : 70 to 70 : 30; 40 : 60 to 60: 40 or be approximately 50 : 50.

### Example 1

Monocyte/macrophage differentiation and functional changes.

Rodent and human monocytes were studied during their differentiation process towards macrophages in the presence or absence of different, physiologically relevant levels of cortisol or corticosterone, as well as aldosterone, respectively.

Glucocorticoid receptor (GR) and mineralocorticoid receptor occupancy was modulated with either eplerenone or ZK91587 or canrenoate (as MR-antagonists) or RU486 (GR-antagonist). Corticosterone (dehydrocorticosterone in rodent cells) was added as a substrate for 11-beta-HSD1. Metyrapone was used to block 11-beta-HSD1 reductase activity. Inhibitors were added with 10⁻⁷ M cortisol, 10⁻⁹ M aldosteron concentration, HSD inhibitors were added in 10⁻⁶ M concentration.

Monocytes were first stimulated with either lipopolysaccharide (LPS) or Tumor-necrosis-factor-alpha (TNF-apha), interferone-gamma (IFN-gamma), interleukin-13 (IL-13) or interleukin-4 (IL-4) before differentiation into macrophages in the presence of above different agonist/antagonist combinations. In order to evaluate the linkage between inflammation and metabolic disease, mature macrophages were also stimulated with fatty acids via the Toll-like-receptor 4.

All conditions unravelled distinct gene-induction profiles, which indicates that the endocrine milieu essentially influences further differentiation of monocytes into macrophage subsets as evaluated by standard RT-PCR or microarray analysis.

Both, aldosterone as well as cortisol/cortisone induced cell proliferation and maturation of monocytes. Although phenotypically not distinguishable, both cell lines showed different gene expression pattern upon stimulation with either LPS, TNF-alpha, IFN-gamma, IL-3 and IL-4, immune complexes or Transforming growth factor-beta (TGF-beta) as evaluated by RT-PCR or microarray analysis. Particularly interesting were the differences in genes affecting PPAR-gamma, MCP-1, iNOS, MIP2, IL-1 and IL-1 receptor antagonist.

Over all, the induction with aldosterone resulted in a more proinflammatory macrophage phenotype as indicated by the induction of nuclear factor kappa-B (NfkappaB), RANK-ligand and upregulation of interleukin-6 and other mediators of tissue destruction during inflammatory processes. This was also reflected by an altered generation of reactive oxygen species (ROS) and plasminogen activator inhibitor-1.

Furthermore, addition of a selective 11-beta-HSD-1 antagonist, which blocks activation of cortisol (corticosterone in rodents), abolished the effects of corticosterone on macrophage differentiation and function.

Particularly important were, however, the effects of metyrapone, which only blocks the activation of corticosterone to cortisol (dehydrocortisone to corticosterone in rodents), affecting macrophage differentiation in as much as the effects of aldosterone at the high affinitity MR were enhanced or were able to override glucocorticoid mediated effects via stimulation of the GR, similar with GR-blockade.

### Example 2

### Differentiation of 3T3-L1 fibroblast to adipocytes.

3T3-L1 fibroblasts represent a prototypic cell line for the maturation and function of adipocytes from progenitor cells. Aldosterone as well as cortisol are able to induce cell proliferation and maturation to pre-adipocytes and further mature adipocytes. Importantly, fibroblasts express 11-beta-HSD-1 in an early stage of differentiation in contrast with monocytes. Furthermore, during adipocyte differentiation 11-beta-HSD-1 switches from the dehydrogenase to the reductase function, i.e. generation of cortisol.

Cell cultures were established as described above, with different combinations of inhibitors and agonists or antagonists of the mineralocorticoid receptor and / HSD enzymes respectively. Differentiation of 3T3-L1-cells to the adipocyte phenotype was evaluated by microscopic evaluation of lipid droplet accumulation, and quantitative measurement of triglycerides and glycerol-3-phosphate dehydrogenase, an enzyme specific for adipocyte differentiation. Cells were stimulated with LPS, TNF-alpha or IL-1 as well as fatty acids.

Similar to the effects described for the monocyte/macropharge lineage from Example 1, the different combinations of ligands/inhibitors unravelled distinct functional patterns. Different to monocytes, the expression of 11-beta-HSD-1 during early differentiation resulted in a proinflammatory phenotype even at the early differentiation stage. Contributing an MR-antagonist fully reversed this phenotype and resulted in anti-adipogenic effects described for 11-beta-HSD-1 inhibition in transgene animal models. The positive effect of MR-blockade in addition to 11-beta-HSD-1 inhibition was evidenced by increased expression of PPAR-gamma and adiponectin. COX-2 expression was reduced by an over all increased glucocorticoid tonus.

### Example 3

### Inhibition of 11-beta-HSD-2: Influences of GR or MR blockade in endothelial cells.

Vascular smooth muscle cells were incubated with varying combinations of a MR or GR-antagonist and physiologically relevant levels of cortisol or aldosterone; importantly, adosterone and cortisol were also used in combination to mimic specific interactions between the ligand-receptor and co-factor recruitment as observed in vitro and in vivo. Aldosterone induced vascular endothelial growth factor (VEGF) as well as ICAM-1; i.e. MR occupancy resulted in enhanced angiogenic factors and recruitment of monocyte/macrophages. Blockade of MR with a selective antagonist, i.e. eplerenone, reversed this effect. Furthermore, inhibition of 11-beta-HSD-2 by glycyrrhetinic acid or siRNA prevented activation of the MR by cortisol.

Further, the following references are hereby incorporated by reference:
1. Wilkinson-Berka JL, Tan G, Jaworski K, Miller AG. Identification of a Retinal Aldosterone System and the Protective Effects of Mineralocorticoid Receptor Antagonism on Retinal Vascular Pathology. Circ. Res. 2009;104(1):124-133.
2. Rickard A, Young M. Corticosteroid receptors, macrophages and cardiovascular disease. J. Mol. Endocrinol. 2009:JME-08-0144.
3. Osmond JM, Dorrance AM. 11{beta}HSD2 Inhibition Causes Cerebrovascular Remodeling and Increases Infarct Size Following Cerebral Ischemia. Endocrinology 2008:en.2008-0808.
4. Odermatt A, Gumy C. Glucocorticoid and mineralocorticoid action: why should we consider influences by environmental chemicals? Biochem Pharmacol 2008;76(10):1184-93.
5. Molnar GA, Lindschau C, Dubrovska G, Mertens PR, Kirsch T, Quinkler M, et al. Glucocorticoid-Related Signaling Effects in Vascular Smooth Muscle Cells. Hypertension 2008;51 (5):1372-1378.
6. Guo C, Ricchiuti V, Lian BQ, Yao TM, Coutinho P, Romero JR, et al. Mineralocorticoid Receptor Blockade Reverses Obesity-Related Changes in Expression of Adiponectin, Peroxisome Proliferator-Activated Receptor-{gamma}, and Proinflammatory Adipokines. Circulation 2008;117(17):2253-2261.
7. Walker BR. Glucocorticoids and Cardiovascular Disease. Eur. J. Endocrinol. 2007;157(5):545-8. Clark AR. Anti-inflammatory functions of glucocorticoid-induced genes. Mol Cell Endocrinol 2007;275(1-2):79-97.
9. Caprio M, Feve B, Claes A, Viengchareun S, Lombes M, Zennaro M-C. Pivotal role of the mineralocorticoid receptor in corticosteroid-induced adipogenesis. FASEB J 2007;21(9):2185-2194.
10. Hultman ML, Krasnoperova NV, Li S, Du S, Xia C, Dietz JD, et al. The Ligand-Dependent Interaction of Mineralocorticoid Receptor with Coactivator and Corepressor Peptides Suggests Multiple Activation Mechanisms. Mol. Endocrinol. 2005;19(6):1460-1473.
11. Chrousos GP, Kino T. Intracellular Glucocorticoid Signaling: A Formerly Simple System Turns Stochastic. Science's STKE 2005;2005(304):pe48-.
12. Walker EA, Stewart PM. 11 beta-hydroxysteroid dehydrogenase: unexpected connections. Trends Endocrinol Metab 2003;14(7):334-9.

## Claims

1. A pharmaceutical composition comprising:
a) a steroid-dehydrogenase-reductase inhibitor (especially an inhibitor of 11-beta-HSD1 or 11-beta-HSD2) and
b) a mineralocorticoid receptor (MR) antagonist.

2. A therapy using a combination of
a) a steroid-dehydrogenase-reductase inhibitor (especially an inhibitor of 11-beta-HSD1 or 11-beta-HSD2) and
b) a mineralocorticoid receptor antagonist.

3. A pharmaceutical composition according to claim 1 or a therapy according to claim 2, using an inhibitor of 11-beta-HSD1 or 11-beta-HSD2 for the prevention of conditions associated with inadequate aldosterone or inadequate cortisol.

4. A pharmaceutical composition according to claim 1 or a therapy according to claim 2, for the treatment and/or prevention of undesired effects of increased amounts of glucocorticoids associated with occupation of MR by cortisol as observed in cardiovascular diseases, stroke, vascular disease of the eye, heart and lung, osteoporosis, rheumatoid arthritis, periodontitis, inflammatory bowel disease, colitis ulcerosa, asthma, chronic obstructive pulmonary disease and inflammatory skin diseases, and cancer related to increased angionesis such as: colon cancer, osteosarcoma, non small cell bronchial carcinoma, breast cancer, renal cell carcinoma, urinary bladder carcinoma, acute lymphoblastic leukemia or endometriosis.

5. A pharmaceutical composition according to claim 1 or a therapy according to claim 2, for the treatment and/or prevention of conditions associated with occupation of MR either by aldosterone or cortisol.

6. A pharmaceutical composition according to claim 1 or a therapy according to claim 2, for the treatment and/or prevention of conditions associated with MR activation such as cardiovascular diseases, stroke, pathologic angiogenesis, metabolic syndrome.

7. A pharmaceutical composition according to claim 1 or a therapy according to claim 2, for the treatment and/or prevention of inflammatory diseases associated with decreased bone formation and/or increased bone destruction; in particular rheumatoid arthritis, periodontal diseases, asthma, inflammatory skin diseases.

8. A pharmaceutical composition according to claim 1 or a therapy according to claim 2, for use to prevent bone destruction in immune- and/or inflammation induced bone loss due to an underlying chronic inflammatory condition, such as osteoporosis, diabetes, colitis ulcerosa.

9. A pharmaceutical composition according to anyone of claims 1 or 3 to 8 or a therapy according to anyone of claims 2 to 8, wherein the 11-beta-HSD reductase inhibitor is selected from BVT116429, Compound 544, 5-chloro-1-(4-tert-butylphenylsulfonyl)-2,3-dihydroindole, 3-(R)-1-(4-tert-butylphenylsulfonyl)-3-imidazol-1-yl)-pyrrolidine, 3-(R)-1-(4-tert-butylphenylsulfonyl)-3-methoxypyrrolidine, 2-(S)-2-methyl-1-(4-tert-butylphenylsulfonyl)-morpholine, syn-2,6-dimethyl-1-(4-(2,2,2-trifluoro-1-hydroxy-1-methylethyl)phenyl-sulfonyl)-piperidine, 1-(4-(2,2,2-trifluoro-1-hydroxy-1-methylethyl)phenylsulfonyl)-homopiperidine, 2-(R)-2-methyl-1-(4-(2,2,2-trifluoro-1-hydroxy-1-methylethyl)phenyl-sulfonyl)-piperidine, 2-(S)-2-methyl-1-(4-(2,2,2-trifluoro-1-hydroxy-1-methylethyl)phenyl-sulfonyl)-piperidine, 2-ethyl-1-(4-(2,2,2-trifluoro-1-hydroxy-1-methylethyl)phenylsulfonyl)-piperidine, 1-(4-(2,2,2-trifluoro-1-hydroxy-1-methylethyl)phenylsulfonyl)-piperidine, 2-(4-(2,2,2-trifluoro-1-hydroxy-1-methylethyl)phenylsulfonyl)-1,2,3,4-tetrahydroisoquinoline, 2-(S)-2-(pyridin-3-yl)-1-(4-(2,2,2-trifluoro-1-hydroxy-1-methylethyl)-phenylsulfonyl)-piperidine, 1-(4-(2,2,2-trifluoro-1-hydroxy-1-methylethyl)phenylsulfonyl)-1,2,3,4-tetrahydroquinoline, 1-(4-(2,2,2-trifluoro-1-hydroxy-1-methylethyl)phenylsulfonyl)-heptamethyleneimine, 3-fluoro-1-(4-(2,2,2-trifluoro-1-hydroxy-1-methylethyl)phenylsulfonyl)-piperidine, 1-(4-(2,2,2-trifluoro-1-hydroxy-1-methylethyl)phenylsulfonyl)-2-(2-imidazol-1-yl-ethyl)piperidine, 2-(2-pyrazol-1-yl-ethyl)-1-(4-(2,2,2-trifluoro-1-hydroxy-1-methylethyl)-phenylsulfonyl)-piperidine, 3-(R)-3-(piperidin-1-yl)-1-(4-(2,2,2-trifluoro-1-hydroxy-1-methylethyl)-phenylsulfonyl)-pyrrolidine and 2-(2-hydroxyethyl)-1-(4-(2,2,2-trifluoro-1-hydroxy-1-methylethyl)-phenylsulfonyl)-piperidine, ((1S)-2-{[1-(phenylthio)cyclopropyl]carbonyl}-1,2,3,4-tetrahydroisoquinolin-1-yl)methanol; 2-{[1-(phenylthio)cyclopropyl]carbonyl}-1,2,3,4-tetrahydroisoquinoline, 6-{[1-(phenylthio)cyclopropyl]carbonyl)}4,5,6,7-tetrahydrothieno[2,3-c]pyridine, 3-phenyl-1-{[1-(phenylthio)cyclopropyl]carbonyl}piperidine, 1'-{[1-(phenylthio)cyclopropyl]carbonyl}-1,3-dihydrospiro[indene-2,4'-piperidine]; 2-methyl-1-phenyl-4-{[1-(phenylthio)cyclopropyl]carbonyl}piperazine, 2-{[1-(phenylthio)cyclopropyl]carbonyl}-2,3,3a,4,5,9b-hexahydro-1H-benzo[e]isoindole, 3-(3-fluorophenyl)-1-{[1-(phenylthio)cyclopropyl]carbonyl}pyrrolidine, 1'-{[1-(phenylthio)cyclopropyl]carbonyl}-3H-spiro[2-benzofuran-1,3'-pyrrolidin]-3-one, ((3S)-2-{[1-(Phenylthio)cyclopropyl]carbonyl}-1,2,3,4-tetrahydroisoquinolin-3-yl)methanol, 2-(4-(Hydroxymethyl)-1-{[1-(phenylthio)cyclopropyl]carbonyl}pyrrolidin-3-yl)phenol, 2-{[1-(Phenylthio)cyclopropyl]carbonyl}-1,2,3,3a,4,9b-hexahydrochromeno[3,4-c]pyrrole, 1'-({1-[(4-Chlorophenyl)thio]cyclopropyl}carbonyl)-3H-spiro[2-benzofuran-1,3'-pyrrolidin]-3-one, 1-{[1-(Cyclohexylsulfonyl)cyclopropyl]carbonyl}pyrrolidine, 4-(1-{[1-(Cyclohexylsulfonyl)cyclopropyl]carbonyl}pyrrolidin-3-yl)pyridine, 4-[1-({1-[(4-Chlorophenyl)thio]cyclopropyl}carbonyl)pyrrolidin-3-yl]pyridine, 1-({1-[(4-Chlorophenyl)thio]cyclopropyl}carbonyl)-3-(3-fluorophenyl)pyrrolidine, 3-(4-Chlorophenyl)-1-({1-[(4-chlorophenyl)thio]cyclopropyl}carbonyl)pyrrolidine, 2-[1-({1-[(4-Chlorophenyl)thio]cyclopropyl}carbonyl)pyrrolidin-3-yl]pyridine, 1'-({1-[(3,5-Dichlorophenyl)thio]cyclopropyl}carbonyl)-3H-spiro[2-benzofuran-1,3'-pyrrolidin]-3-one, 1'-({1-[(3-Chloro-4-fluorophenyl)thio]cyclopropyl}carbonyl)-3H-spiro[2-benzofuran-1,3'-pyrrolidin]-3-one, 1'-({1-[(2,6-Dichlorophenyl)thio]cyclopropyl}carbonyl)-3H-spiro[2-benzofuran-1,3'-pyrrolidin]-3-one, 1'-({1-[(4-Fluorophenyl)thio]cyclopropyl}carbonyl)-3H-spiro[2-benzofuran-1,3'-pyrrolidin]-3-one, 1'-({1-[(4'-Fluorobiphenyl-4-yl)thio]cyclopropyl}carbonyl)-3H-spiro[2-benzofuran-1,3'-pyrrolidin]-3-one, 1'-({1-[(3,4-Dichlorophenyl)thio]cyclopropyl}carbonyl)-3H-spiro[2-benzofuran-1,3'-pyrrolidin]-3-one, 1'-[(1-{[3-(Trifluoromethyl)phenyl]thio}cyclopropyl)carbonyl]-3H-spiro[2-benzofuran-1,3'-pyrrolidin]-3-one, 1'-[(1-{[4-(Trifluoromethoxy)phenyl]thio}cyclopropyl)carbonyl]-3H-spiro[2-benzofuran-1,3'-pyrrolidin]-3-one, 1'-({1-[(4-Chlorophenyl)thio]cyclopropyl}carbonyl)-3 H-spiro[2-benzofuran-1,3'-pyrrolidine], 1'-({1-[(4'-Fluorobiphenyl-4-yl)thio]cyclopropyl}carbonyl)-3H-spiro[2-benzofuran-1,3'-pyrrolidine], 1-{[1-(Cyclohexylsulfonyl)cyclopropyl]carbonyl}-3-phenylpiperazine, 1-({1-[(4-Chlorophenyl)thio]cyclopropyl}carbonyl)-3-phenylpiperazine, 6-{[1-(Phenylthio)cyclopropyl]carbonyl}-3a,4,5,6,7,7a-hexahydrothieno[2,3-c]pyridine, (4aR,8aS)-2-({1-[(4-Chlorophenyl)thio]cyclopropyl}carbonyl)decahydroisoquinoline, 1-({1-[(4-Chlorophenyl)thio]cyclopropyl}carbonyl)decahydroquinoline, 3-Chloro-2-methyl-N-(2-methyl-pyrimidin-4-yl)-benzenesulfonamide, 3-Chloro-N-(2-cyclopropyl-pyrimidin-4-yl)-2-methyl-benzenesulfonamide, N-(2-Cyclopropyl-pyrimidin-4-yl)-2,5-difluoro-benzenesulfonamide, Naphthalene-2-sulfonic acid (2-cyclopropyl-pyrimidin-4-yl)-amide, Biphenyl-4-sulfonic acid (2-cyclopropyl-pyrimidin-4-yl)-amide, Quinoline-8-sulfonic acid (2-cyclopropyl-pyrimidin-4-yl)-amide, N-(2-Cyclopropyl-pyrimidin-4-yl)-benzenesulfonamide, N-(2-Cyclopropyl-pyrimidin-4-yl)-5-fluoro-2-methyl-benzenesulfonamide, N-(2-Cyclopropyl-pyrimidin-4-yl)-3-methoxy-benzenesulfonamide, N-(2-Cyclopropyl-pyrimidin-4-yl)-2-methoxy-5-methyl-benzenesulfonamide, 3-Chloro-N-(2-cyclopropyl-pyrimidin-4-yl)-4-methoxy-benzenesulfonamide, 5-Chloro-N-(2-cyclopropyl-pyrimidin-4-yl)-2-methoxy-benzenesulfonamide, 5-Fluoro-N-(2-isopropyl-pyrimidin-4-yl )-2-methyl-benzenesulfonamide, 3,4-Dichloro-N-(2-isopropyl-pyrimidin-4-yl)-benzenesulfonamide, N-(2-lsopropyl-pyrimidin-4-yl)-4-(1.3-oxazol-5-yl)-benzenesulfonamide, 2,4-Dichloro-N-(2-isopropyl-pyrimidin-4-yl)-6-methyl-benzenesulfonamide, 2,3-Dichloro-N-(2-isopropyl-pyrimidin-4-yl)-benzenesulfonamide, 4,5-Dichloro-thiophene-2-sulfonic acid (2-isopropyl-pyrimidin-4-yl)-amide, 5-Pyridin-2-yl-thiophene-2-sulfonic acid (2-isopropyl-pyrimidin-4-yl)-amide, 3-Chloro-N-(2-isopropyl-pyrimidin-4-yl)-2-methyl-benzenesulfonamide, N-(2-Isopropyl-pyrimidin-4-yl)-3-trifluoromethyl-benzenesulfonamide, N-(2-Isopropyl-pyrimidin-4-yl)-2-trifluoromethyl-benzenesulfonamide, 5-Chloro-thiophene-2-sulfonic acid (2-isopropyl-pyrimidin-4-yl)-amide, N-(2Isopropyl-pyrimidin-4-yl)-4-trifluoromethyl-benzenesulfonamide, Piperidine-1 -sulfonic acid (2-isopropyl-pyrimidin-4-yl)-amide, Naphthalene-2-sulfonic acid (2-isopropyl-pyrimidin-4-yl)-amide, Biphenyl-4-sulfonic acid (2-isopropyl-pyrimidin-4-yl)-amide, 2,5-Difluoro-N-(2-isopropyl-pyrimidin-4-yl)-benzenesulfonamide, N-(2-Isopropyl-pyrimidin-4-yl)-3,4-dimethoxy-benzenesulfonamide, N-(2-tert-Butyl-pyrimidin-4-yl )-3,4-dichloro-benzenesulfonamide, N-(2-tert-Butyl-pyrimidin-4-yl)-5-fluoro-2-methyl-benzenesulfonamide, Naphthalene-2-sulfonic acid (2-tert-butyl-pyrimidin-4-yl)-amide, N-(2-tert-Butyl-pyrimidin-4-yl)-2,5-difluoro-benzenesulfonamide, N-(2-tert-Butyl-pyrimidin-4-yl)-4-(1.3-oxazol-5-yl)-benzenesulfonamide, 3-Chloro-N-(2-ethyl-pyrimidin-4-yl)-2-methyl-benzenesulfonamide, 2,4-Dichloro-N-(2-ethyl-pyrimidin-4-yl)-6-methyl-benzenesulfonamide, 4-Chloro-N-(2-ethyl-pyrimidin-4-yl )-2,5-dimethyl-benzenesulfonamide, 3-Chloro-N-(2-cyclobutyl-pyrimidin-4-yl)-2-methyl-benzenesulfonamide, Naphthalene-2-sulfonic acid (2-cyclobutyl-pyrimidin-4-yl)-amide, 5-Pyridin-2-yl-thiophene-2-sulfonic acid (2-cyclobutyl-pyrimidin-4-yl)-amide, 2,4-Dichloro-N-(2-cyclobutyl-pyrimid in-4-yl)-6-methyl-benzenesulfonamide, 3,4-Dichloro-N-(2-methoxymethyl-pyrimidin-4-yl)-benzenesulfonamide, 3-Chloro-N-(2-cyclopropylmethoxymethyl-pyrimidin-4-yl)-2-methyl-benzenesulfonamide, 3-Chloro-2-methyl-N-(2-morpholin-4-ylmethyl-pyrimidin-4-yl)-benzenesulfonamide, Naphthalene-2-sulfonic acid (2,6-dimethyl-pyrimidin-4-yl)-amide, 3-Chloro-N-(2-cyclopropyl-pyrimidin-4-yl)-2-N-dimethyl-benzenesulfonamide, 3,4-Dichloro-N-(2-isopropyl-pyrimidin-4-yl)-N-methyl-benzenesulfonamide, 3-Chloro-2-methyl-N-(2-phenyl-pyrimidin-4-yl)-benzenesulfonamide, 3-Chloro-2-methyl-N-[2-(2-methyl-thiazol-4-yl)-pyrimidin-4-yl]-benzenesulfonamide, BNW7, lithocholic acid, lithocholic acid, carbenoxolone, glycyrrhetinic acid, and glycyrrhetinic acid derivatives

10. A pharmaceutical composition according to anyone of claims 1 or 3 to 9 or a therapy according to anyone of claims 2 to 9, wherein the MR-antagonist is selected from RU28328, Spironolactone, (E)- and (Z)-Guggulsterone, Eplerenone, Canrenoate, Fludrocortisone, Fluticasone propionate, Miconazole, Mometasone furoate (Sch 32088), R1881, 2-(3-chloro-4-cyanophenyl)-3-(4-fluorophenyl)-3,3a,4,5-tetrahydro-2H-benzo[g]indazole-7-carboxylic acid, 3-(4-fluorophenyl)-7-hydroxy-3,3a,4,5-tetrahydrobenzo[g]indazol-2-yl)-2-methylbenzonitrile, 3-(4-fluorophenyl)-7-hydroxy-3,3a,4,5-tetrahydrobenzo[g]indazol-2-yl)-2-trifluoromethyl-benzonitrile, 2-chloro-4-(3-(4-fluorophenyl)-7-hydroxy-3,3a,4,5-tetrahydrobenzo[g]indazol-2-yl)benzonitrile, 2-(3-chloro-4-cyanophenyl)-3-(4-fluorophenyl)-N-(2-(methylsulfonyl)ethyl)-3,3a,4,5-tetrahydro-2H-benzo[g]indazole-7-carboxamide, 2-(3-chloro-4-cyanophenyl)-3-cyclopentyl-3,3a,4,5-tetrahydro-2H-benzo[g]indazole-7-carboxylic acid, 2-(4-cyanophenyl)-3-cyclopentyl-3,3a,4,5-tetrahydro-2H-benzo[g]indazole-7-carboxylic acid, 2-(4-cyano-3-methylphenyl)-3-cyclopentyl-3,3a,4,5-tetrahydro-2H-benzo[g]indazole-7-carboxylic acid, 2-(5-cyano-6-methylpyridin-2-yl)-3-cyclopentyl-3,3a,4,5-tetrahydro-2H-benzo[g]indazole-7-carboxylic acid, 2-(4-cyano-3-methoxyphenyl)-3-cyclopentyl-3,3a,4,5-tetrahydro-2H-benzo[g]indazole-7-carboxylic acid, N-(2-(3-chloro-4-cyanophenyl)-3-cyclopentyl-3,3a,4,5-tetrahydro-2H-benzp[g]indazol-7-yl)-acetamide, methyl 2-(3-chloro-4-cyanophenyl)-3-cyclopentyl-3,3a,4,5-tetrahydro-2H-benzo[g]indazole-7-carboxylate, 2-(3-chloro-4-cyanophenyl)-3-cyclopentyl-3a-methyl-3,3a,4,5-tetrahydro-2H-benzo[g]indazole-7-carboxylic acid, 2-(3-chloro-4-cyanophenyl)-3cyclopentyl-N-(2-(methylsulfonyl)ethyl)-3,3a,4,5-tetrahydro-2H-benzo[g]indazole-7-carboxamide, 2-(3-chloro-4-cyanophenyl)-3-cyclopentyl-N-(2-hydroxyethyl)-3,3a,4,5-tetrahydro-2H-benzo[g]indazole-7-carboxamide, 2-(4-cyano-3-methylphenyl)-3-cyclopentyl-2,3,3a,4-tetrahydro-chromeno[4:3c]pyrazole-7-carboxylic acid, 2-(3-chloro-4-cyanophenyl)-3-cyclobutyl-3,3a,4,5-tetrahydro-2H-benzo[g]indazole-7-carboxylic acid, 2-(3-chloro-4-cyanophenyl)-3-cyclopentenyl-3,3a,4,5-tetrahydro-2H-benzo[g]indazole-7-carboxylic acid, 2-(4-cyano-3-methylphenyl)-3-cyclopentenyl-3,3a,4,5-tetrahydro-2H-benzo[g]indazole-7-carboxylic acid, 2-(3-chloro-4-cyanophenyl)-3-(5-methyl-2-furyl)-3,3a,4,5-tetrahydro-2H-benzo[g]indazole-7-carboxylic acid, 2-(3-chloro-4-cyanophenyl)-3-(3-furyl)-3,3a,4,5-tetrahydro-2H-benzo[g]indazole-7-carboxylic acid, 2-(3-chloro-4-cyanophenyl)-3-(5-methyl-2-furyl)-N-[2-(methylsulfonyl)ethyl]-3,3a,4,5-tetrahydro-2H-benzo[g]indazole-7-carboxamide, 2-(3-chloro-4-cyanophenyl)-N-[2-(methylsulfonyl)ethyl]-3-(2-methyl-1,3-thiazol-5-yl)-3,3a,4,5-tetrahydro-2H-benzo[g]indazole-7-carboxamide, 2-(3-chloro-4-cyanophenyl)-3-cyclopentyl-2,3,3a,4-tetrahydrochromeno[4:3c]pyrazole-7-carboxylic acid, 2-[4-cyano-3-(trifluorornethyl)phenyl]-3-cyclopentyl-2,3,3a,4-tetrahydro-chromeno[4,3c]pyrazole-7-carboxylic acid, 2-(4-cyano-3-methylphenyl)-3-cyclopentenyl-2,3,3a,4-tetrahydrochromeno[4:3c]pyrazole-7-carboxylic acid, and 2-(3-chloro-4-cyanophenyl)-3-cyclopentenyl-2,3,3a,4-tetrahydrochromeno[4:3c]pyrazole-7-carboxylic acid, 2-(3-chloro-4-cyanophenyl)-3-cyclopentyl-3,3a,4,5-tetrahydro-2H-benzo[g]indazole-7-carboxylic acid, 2-(4-cyano-3-methylphenyl)-3-cyclopentyl-3,3a,4,5-tetrahydro-2H-benzo[g]indazole-7-carboxylic acid, or 2-(4-cyano-3-methoxyphenyl)-3-cyclopentyl-3,3a,4,5-tetrahydro-2H-benzo[g]indazole-7-carboxylic acid.

11. A pharmaceutical composition comprising:
a) carbenoxolone, glycyrrhetinic acid, a glycyrrhetinic acid derivative, lithocholic acid or a lithocholic acid derivative, and
b) eplerenone.

12. A therapy using a combination of
a) carbenoxolone, glycyrrhetinic acid, a glycyrrhetinic acid derivative, lithocholic acid or a lithocholic acid derivative, and
b) eplerenone.

13. A kit of parts comprising:
a) a steroid-dehydrogenase-reductase inhibitor (especially an inhibitor of 11-beta-HSD1 or 11-beta-HSD2) and
b) a mineralocorticoid receptor antagonist.
